**Europäisches Patentamt**

**European Patent Office**

**Office européen des brevets**

(19)

(11) Veröffentlichungsnummer: **0 158 254**
**B1**

(12) **EUROPÄISCHE PATENTSCHRIFT**

(45) Veröffentlichungstag der Patentschrift:
08.06.88

(21) Anmeldenummer: **85103896.8**

(22) Anmeldetag: **01.04.85**

(51) Int. Cl.⁴: **C 12 Q 1/56** //
**G01N33/86**

(54) **Reagenz zur Bestimmung der Thromboplastinzeit.**

(30) Priorität: **09.04.84 DE 3413311**

(43) Veröffentlichungstag der Anmeldung:
**16.10.85 Patentblatt 85/42**

(45) Bekanntmachung des Hinweises auf die Patenterteilung:
**08.06.88 Patentblatt 88/23**

(84) Benannte Vertragsstaaten:
**AT BE CH DE FR GB IT LI LU NL SE**

(56) Entgegenhaltungen:
**EP-A-0 003 474**
**EP-A-0 014 039**
**EP-A-0 062 310**
**GB-A-917 012**
**US-A-4 169 015**

(73) Patentinhaber: **BEHRINGWERKE AKTIENGESELLSCHAFT, Postfach 1140, D-3550 Marburg/Lahn (DE)**

(72) Erfinder: **Kolde, Hans- Jürgen, Dr., Höhenweg 54, D-3550 Marburg 1 (DE)**

(74) Vertreter: **Becker, Heinrich Karl Engelbert, Dr., HOECHST AKTIENGESELLSCHAFT Central Patent Department P.O. Box 80 03 20, D-6230 Frankfurt am Main 80 (DE)**

**Beschreibung**

Die Erfindung betrifft ein Reagenz zur Bestimmung der Thromboplastinzeit mittels einem chromogenen Substrat für Thrombin.

Die Bestimmung der Thromboplastinzeit (TPZ) gehört zu den am häufigsten durchgeführten Gerinnungstesten. Durch den Einsatz der TPZ für die Überwachung der oralen Antikoagulantientherapie mit Cumarin-Derivaten gewann dieser Test zunehmend an Bedeutung, insbesondere durch die Tatsache, daß der Erfolg einer Therapie entscheidend von der Überwachung der Einhaltung des therapeutischen Bereiches abhängt.

Bei der TPZ-Bestimmung wird die Plasma- oder Blutprobe mit einem Gewebethromboplastin aus tierischem oder menschlichem Gewebe aktiviert. Das Gewebethromboplastin aktiviert den Faktor VII des exogenen Gerinnungssystems, der über die Aktivierung von Faktor X aus Prothrombin Thrombin freisetzt. In der konservativen Gerinnungsanalytik erfolgt dann die Thrombinbestimmung über die Gerinnungszeitmessung mit dem natürlichen Substrat Fibrinogen. Von entscheidender Bedeutung ist es, daß das Gewebethromboplastin eine hohe Empfindlichkeit für alle Vitamin K-abhängigen Gerinnungsfaktoren des exogenen Gerinnungsweges II, VII und X aufweist, da diese durch die Cumarin-Therapie erniedrigt werden. Das als internationaler Standard akzeptierte British Comperative Thromboplastin erfaßt diese Faktoren mit hoher Empfindlichkeit.

Zur Überwachung der Antikoagulantientherapie werden auch sogenannte II-, VII- oder X-Reagenzien verwendet. Diesen Thromboplastinreagenzien wird Faktor V und Fibrinogen zugesetzt, um Schwankungen im Gehalt dieser Faktoren auszuschließen. Man erreicht dadurch, daß Plasma- oder Blutproben auch eine gewisse Zeit gelagert werden können, ehe sie untersucht werden, da der Faktor V - der die TPZ entscheidend beeinflußt - schnell an Aktivität verliert.

Da der Faktor VII die kürzeste biologische Halbwertszeit hat, spricht er am schnellsten auf die Therapie mit Cumarin-Derivaten an. Daher gelten Faktor VII-empfindliche Thromboplastine als besonders geeignet, auch die Einstellphase eines Patienten zu überwachen. Die Kontrolle von Faktor X in der Einstellphase ist ebenfalls indiziert (Aiach, M. et al., 1982, Thromb.Res. 47, 69-71), so daß auch eine Faktor-X-Empfindlichkeit anzustreben ist.

Die Entwicklung von niedermolekularen chromogenen Peptidsubstraten für die aktivierten Faktoren der Gerinnungskaskade ermöglicht es seit einiger Zeit, das Fibrinogen als klassisches Gerinnungssubstrat zu ersetzen. So gibt es heute relativ spezifische Substrate für die Vitamin-K-abhängigen Faktoren X und II. Es wurden empfindliche Methoden zur Bestimmung der Faktoren X, VII und des F II im Plasma entwickelt, und es gelang, relativ günstige Korrelationen zwischen der TPZ-Bestimmung und den Einzelfaktorbestimmungen zu erzielen. Nachteil all dieser Einzelfaktorbestimmungen ist es, daß sie nur einen der Vitamin-K-abhängigen Faktoren erfassen und daher nicht die pathophysiologische Situation des Patienten widerspiegeln, bei dem alle 3 Faktoren erniedrigt sind. Insbesondere kann der kritische Faktor VII auch heute nicht direkt mit einem spezifischen chromogenen Substrat, sondern nur über den Faktor X gemessen werden.

In der EP-A-0 014 039 wurde ein Verfahren beschrieben, das die TPZ mit Hilfe eines chromogenen Substrates bestimmt. Um zu vernünftigen Meßzeiten zu gelangen, ist allerdings ein sogenanntes Serumreagenz notwendig, welches aus recalcifiziertem Plasma durch Thromboplastin-Aktivierung erhalten wird. Die beschriebene Aufarbeitung des Serums garantiert erfahrungsgemäß nicht die restlose Entfernung aller Faktoren, insbesondere nicht der des exogenen Weges der Gerinnung. Ein Thromboplastin, das in Verbindung mit einem Serum verwendet wird, welches in der beschriebenen Weise hergestellt wurde, kann daher keine hohe Faktorenabhängigkeit aufweisen, insbesondere dann nicht, wenn - wie in der Patentschrift vorgeschlagen - dem Reagenz direkt Thrombin zugesetzt wird.

In der DE-A-31 13 350 wurden ein weiteres Verfahren und Reagenz vorgestellt, mit chromogenem Substrat die Thromboplastinzeit zu messen. Um zu einem ausreichend stabilen Reagenz zu kommen, ist es erforderlich, ein besonders hergestelltes Thromboplastin zu verwenden. Das in dieser Anmeldung benutzte Thrombinsubstrat unterliegt nämlich einer unspezifischen Hydrolyse bereits durch im Thromboplastin enthaltene Proteasen. Durch das modifizierte Herstellungsverfahren für das Thromboplastin wurden jedoch diese Proteasen weitgehend entfernt so daß das Reagenz eine wesentlich bessere Stabilität zeigt. Aus Gründen der Vergleichbarkeit von fotometrischen mit koagulometrischen Methoden zur Bestimmung der Thromboplastinzeit ist es jedoch wünschenswert, beide Reagenzientypen aus dem gleichen Thrombplastin herzustellen. Außerdem kann man aus einer Aufarbeitung von Thromboplastin dann gleichermaßen ein fotometrisches und ein koagulometrisches Reagenz gewinnen.

Überraschenderweise läßt sich ein solches Reagenz mit herkömmlichen Thromboplastinen herstellen, wenn man ein chromogenes Substrat mit höherer Spezifität für Thrombin einsetzt, beispielsweise H-D-Phe-Pro-Arg-ANBS-isopropylamid oder andere ANBS-Derivate wie ANBS-Glycinethylester oder ANBS-Neopentylamid. Ein solches Reagenz ist Gegenstand der Erfindung.

Tabelle 1 zeigt das Ergebnis der Hydrolyse von chromogenen Substraten für unterschiedliche Proteasen durch Thromboplastinpräparate des Standes der Technik. Durch Messung der Extinktion bei 405 nm erkennt man, daß Thrombinsubstrate der Struktur H-D-Phe-Pro-Arg-ANBS-R, Tos-Gly-Pro-Arg-ANBS-R oder Boc-Gly-Pro-Arg-ANBS-R durch die Thromboplastine nur ' nwesentlich umgesetzt werden, während man bei den meisten anderen Substraten und auch dem in der   E 31 13 350 A1 benützten Substrat für Thrombin, Tos-Gly-Pro-Arg-pNA, einen raschen Verbrauch des Substrates registriert. Alle Thromboplastine zeigen also eine hohe proteolytische Aktivität. In der Praxis bedeutet das, daß ein solches Reagenz mit einem Substrat wie Tos-Gly-Pro-Arg-pNA nur für sehr kurze Zeit haltbar ist, da es zu einer raschen Verminderung der eingesetzten Substratmenge kommt. Ein Reagenz mit beispielsweise H-D-Phe-Pro-Arg-ANBS-isopropylamid ist dagegen wesentlich länger haltbar und seine Haltbarkeit ist durch die Stabilität des Thromboplastins, nicht aber durch die Hydrolyse des Substrates begrenzt.

Die Eigenschaften eines solchen Reagenzes sind daher gut mit einem Gerinnungsreagenz mit dem gleichen Thromboplastin vergleichbar.

Gegenstand der Erfindung ist daher ein Reagenz zur fotometrischen Bestimmung der Thromboplastinzeit, mindestens enthaltend ein proteolytisch wirksames Thromboplastin und ein chromogenes Substrat für Thrombin der Struktur Tos-Gly-Pro-Arg-ANBS-R oder H-D-Phe-Pro-Arg-ANBSR, wobei R $NH-C_nH2_{n1}$ mit $n = 1$ bis 7, NH-Benzyl oder der Rest einer über die alpha-Aminogruppe gebundenen Aminosäure oder deren Alkylester ist, und wobei das chromogene Substrat durch das proteolytisch wirksame Thromboplastin in wässriger Suspension in Gegenwart von Calciumionen zu weniger als 0,00125 mal der Anfangskonzentration pro Stunde bei 37° C umgesetzt wird.

Vorzugsweise wird das Thromboplastin aus menschlicher Plazenta gewonnen.

Bevorzugt wird ein chromogenes Substrat, das die Struktur Tos-Gly-Pro-Arg-ANBS-R oder H-D-Phe-Pro-Arg-ANBS-R hat, wobei R $NH-C_nH_{2n+1}$ mit $n = 1$ bis 7, NH-Benzyl oder der Rest einer über die alpha-Aminogruppe gebundenen Aminosäure oder deren Alkylester ist, verwendet.

Das beschriebene Reagenz kann Calciumionen in einer Konzentration von 0,5 bis 10 mmol/l, vorzugsweise 5 mmol/l, enthalten.

Weiterhin kann es eine Pufferlösung wie Tris, Hepes, Glycyl-Glycin, Epps, Imidazol, bevorzugt Hepes, in einer Konzentration von 0,005 bis 0,1 mol/l, bevorzugt 0,025 mol/l, mit einem pH-Bereich von 7 bis 8,5, bevorzugt 7,6, enthalten.

Es können auch Neutralsalze, vorzugsweise Kochsalz, in einer Konzentration von 0,01 bis 0,2 mol/l, bevorzugt 0,05 mol/l, zugesetzt werden.

Ein solches Reagenz kann auch einen Heparininhibitor wie Protaminsalze oder ®Polybren, vor allem Polybren, in einer Konzentration von 0,01 bis 0,5 mg/l, bevorzugt 0,25 mg/l, enthalten.

Dem Reagenz kann weiterhin menschlicher oder tierischer Faktor V zugesetzt werden.

Das beschriebene Reagenz kann unter Verwendung eines Mangelplasmas für die Faktoren II, VII, X und V zur Bestimmung der Faktoren II, VII, X und V oder auch unter Verwendung eines Mangelplasmas für die Faktoren II, VII und X zur Bestimmung der Faktoren II, VII und X verwendet werden.

Erläuterung der verwendeten Abkürzungen:

| | |
|---|---|
| ANBS | 5-Amino-2-nitro-benzoesäure |
| Phe | Phenylalanin |
| Pro | Prolin |
| Arg | Arginin |
| Tos | Toluolsulfonyl- |
| Gly | Glycin |
| Tris | Trishydroxymethylaminomethan |
| Hepes | N-(2-hydroxyethyl)piperazin-N'-2-ethansulfonsäure |
| Epps | 4-(-2-hydroxyethyl)-1-piperazinpropansulfonsäure |
| Leu | Leucin |
| pNa | para-Nitroanilin |
| Suc | Succinyl- |
| Ala | Alanin |

**Tabelle 1:** Hydrolyse von verschiedenen chromogenen Substraten durch Thromboplastine des Standes der Technik

| Chromogenes Substrat | Thromboplastin | | | | | | |
|---|---|---|---|---|---|---|---|
| | A | B | C | D | E | F | G |
| D-Phe-Pro-Arg-ANBS-isopropylamid | 0,006 | 0,016 | 0,010 | 0,013 | 0,011 | 0 | 0 |
| D-Phe-Pro-Arg-ANBS-Valin-methylester | 0,003 | 0,031 | 0,008 | 0,020 | 0 | 0 | 0,002 |
| D-Phe-Pro-Arg-ANBS-neopentylamid | 0,003 | 0,009 | 0,011 | 0,007 | 0,014 | 0,003 | 0,002 |
| D-Phe-Pro-Arg-ANBS-Glycin-ethylester | 0,007 | 0,009 | 0,015 | 0,019 | 0,008 | 0,011 | 0,003 |
| D-Phe-Pro-Arg-ANBS-n-Butyl-amid | 0,021 | 0,023 | 0,019 | 0,013 | 0,030 | 0,023 | 0,012 |
| Tos-Gly-Pro-Arg-ANBS-iso-propylamid | 0,035 | 0,039 | 0,030 | 0,023 | 0,041 | 0,029 | 0,018 |
| Tos-Gly-Pro-Arg-ANBS-Gly-cinethylester | 0,029 | 0,034 | 0,028 | 0,026 | 0,037 | 0,023 | 0,029 |
| Tos-Gly-Pro-Arg-pNA | 0,190 | 0,143 | 0,115 | 0,137 | 0,123 | 0,116 | 0,149 |
| Leu-pNA | 0,974 | 0,681 | 0,721 | 0,593 | 0,871 | 0,536 | 1,124 |
| Suc-(Ala)$_3$-pNA | 0,211 | 0,324 | 0,248 | 0,411 | 0,178 | 0,310 | 0,202 |
| N--Benzoyl-L-Lysin-pNA | 0,139 | 0,525 | 0,433 | 0,402 | 0,372 | 0,198 | 0,503 |

Extinktionsdifferenz eines nach Beispiel 1 hergestellten Reagenzes nach 92 Stunden bei Raumtemperatur. Die Substratkonzentration betrug 50 mol/l, die Thromboplastine wurden nach Herstellerangaben gelöst und in einer Konzentration von 5 % zugesetzt.

Die folgenden Beispiele sollen die Erfindung weiter erläutern:

**Beispiel 1**

Herstellung eines Reagenzes zur Bestimmung der Thromboplastinzeit mit einem chromogenen Substrat

70 ml einer wässrigen Lösung mit 5,844 g NaCl, 1,47 g CaCl$_2$, 11,92 g HEPES, 250 µg ®Polybren und 67,1 mg H-D-Phe-Pro-Arg-ANBS-isopropylamid-HCl in einem Liter, pH 7,6, wurden mit 4 bis 20 ml humanem Thromboplastin versetzt und lyophilisiert.

Je nach Menge und Aktivität des zugesetzten Thromboplastins erhielt man nach Rekonstitution mit 200 ml Wasser eine Zeit für eine Extinktionsänderung bei 405 nm von 0,1 von 20 bis 40 sec bei einem Normalplasma.

**Beispiel 2**

1. Bezugskurve

Ansatz des Reagenzes analog Beispiel 1. Aus Plasma wird mit physiologischer Kochsalzlösung eine Verdünnungsreihe angesetzt und fotometrisch die Thromboplastinzeit gemessen.

50 µl Plasma oder verdünntes Plama wurden in einer vorgewärmten Kunstoffküvette mit 500 µl Reagenz versetzt und die Extinktion bei 405 nm in Abhängigkeit von der Zeit aufgezeichnet. Aus dem Diagramm wird die Zeit bestimmt, in der eine Extinktionszunahme von 0,1 erreicht ist. Diese Zeit wird gegen die reziproke Konzentration des Plasmas aufgetragen. Man erhält analog zur Gerinnungsmethode eine Gerade zwischen 100 und 5 % der Norm. Der Normalwert des unverdünnten Plasmas beträgt 29,4 Sekunden. Für pathologische Plasmen sind die Zeiten jeweils wesentlich länger.

2.Faktorenempfindlichkeit

Die Empfindlichkeit eines Reagenzes nach Beispiel 1 für die Faktoren II, VII, X und V zeigt Tabelle 2. Plasma wurde mit entsprechenden Mangelplasmen ausverdünnt und wie unter 1 analysiert.

**Tabelle 2:**

| Faktor | Prothrombinratio Gehalt des Faktors in % der Norm | | |
|---|---|---|---|
| | 100 | 25 | 10 |
| X | 1 | 1,35 | 1,75 |
| VII | 1 | 1,2 | 5,8 |
| II | 1 | 1,2 | 1,56 |
| V | 1 | 1,75 | 3,65 |

Man erkennt in Tabelle 2, daß das Reagenz auf eine Verminderung der aufgeführten Faktoren empfindlich anspricht.

**Patentansprüche**

1. Reagenz zur fotometrischen Bestimmung der Thromboplastinzeit, mindestens enthaltend ein proteolytisch wirksames Thromboplastin und ein chromogenes Substrat für Thrombin der Struktur H-D-Phe-Pro-Arg-ANBS-R oder Tos-Gly-Pro-Arg-ANBS-R, wobei R $NH-C_nH_{2n+1}$ mit n = 1 bis 7, NH-Benzyl oder der Rest einer über die alpha-Aminogruppe gebundenen Aminosäure oder deren Alkylester ist, und wobei das Substrat durch das Thromboplastin in wässriger Suspension in Gegenwart von Calciumionen zu weniger als 0,00125 mal der Anfangskonzentration pro Stunde bei 37°C umgesetzt wird.

2. Reagenz nach Anspruch 1, dadurch gekennzeichnet, daß das Thromboplastin aus menschlicher Plazenta gewonnen ist.

3. Reagenz nach Anspruch 1, dadurch gekennzeichnet, daß das Reagenz Calciumionen in einer Konzentration von 0,5 bis 10 mmol/l, vorzugsweise 5 mmol/l enthält.

4. Reagenz nach Anspruch 1, dadurch gekennzeichnet, daß es eine Pufferlösung, vorzugsweise Tris, Hepes, Glycyl-Glycin, Epps, Imidazol, bevorzugt Hepes, in einer Konzentration von 0,005 bis 0,1 mol/l, bevorzugt 0,025 mol/l, mit einem pH-Bereich von 7 bis 8,5, bevorzugt 7,6, enthält.

5. Reagenz nach Anspruch 1, dadurch gekennzeichnet, daß es ein Neutralsalz, vorzugsweise Kochsalz, in einer Konzentration von 0,01 bis 0,2 mol/l, bevorzugt 0,05 mol/l, enthält.

6. Reagenz nach Anspruch 1, dadurch gekennzeichnet, daß das Reagenz ®Polybren (Hexadimethrin bromid/Poly-N,N,N',N'-Trimethyl-N-trimethylen hexamethylen, diammonium dibromid) in einer Konzentration von 0,01 bis 0,5 mg/l, bevorzugt 0,25 mg/l, enthält.

7. Reagenz nach Anspruch 1, dadurch gekennzeichnet, daß es zugesetzten menschlichen oder tierischen Faktor V enthält.

8. Verwendung eines Reagenzes nach Anspruch 1 unter Verwendung eines Mangelplasmas für die Faktoren II, VII, X und V zur Bestimmung der Faktoren II, VII, X und V.

9. Verwendung eines Reagenzes nach Anspruch 7 unter Verwendung eines Mangelplasmas für die Faktoren II, VII und X zur Bestimmung der Faktoren II, VII und X.

10. Verfahren zur Herstellung eines Reagenzes zur photometrischen Bestimmung der Thromboplastinzeit, enthaltend ein proteolytisch wirksames Thromboplastin und ein chromogenes Substrat für Thrombin der Struktur -H-D-Phe-Pro-Arg-ANBS-R oder Tos-Gly-Pro-Arg-ANBS-R, wobei R $NH-C_nH_{2n+1}$ mit n = 1 bis 7, NH-Benzyl oder der Rest einer über die alpha-Aminogruppe gebundenen Aminosäure oder deren Alkylester ist, dadurch gekennzeichnet, dass zu einer Pufferlösung mit einem pH-Bereich von 7 - 8,5 dieses chromogene Substrat hunzugefügt wird, wobei das Substrat durch das Thromboplastin in wässriges Suspension in Gegenwart von Calciumionen zu weniger als 0,00125 mal der Anfangskonzentration pro Stunde bei 37°C umgesetzt wird, sowie gegebenenfalls ein lösliches Kalziumsalz in einer Konzentration von 0,5-10 mmol/l zugegeben und gegebenenfalls lyophilisiert wird.

11. Verfahren zur photometrischen Bestimmung der Thromboplastinzeit in Plasma, dadurch gekennzeichnet, daß eine Verdünnungsreihe des Plasmas bereitet, mit einer Lösung, die ein proteolytisch wirksames Thromboplastin, ein chromogenes Substrat für Thrombin der Struktur H-D-Phe-Pro-Arg-ANBS-R oder Tos-Gly-Pro-Arg-ANBS-R, wobei R NH-$C_nH_{2n+1}$ mit n=1 bis 7, NH-Benzyl oder der Rest einer über die alpha-Aminogruppe gebundenen Aminosäure oder deren Alkylester ist, und wobei das Substrat durch das Thromboplastin in wässriger Suspension in Gegenwart von Calciumionen zu weniger als 0,00125 mal der Anfangskonzentration pro Stunde bei 37°C umgesetzt wird, und gegebenenfalls Calciumionen in einer Konzentration von 0,5-10 mmol/l enthält, versetzt und die Zeit bestimmt wird, in welcher eine bestimmte Extinktionszunahme erreicht wird.

12. Verfahren nach Anspruch 11, dadurch gekennzeichnet, daß das Plasma mit einem Mangelplasma verdünnt wird.

## Claims

1. A reagent for the photometric determination of the prothrombin time, containing, at the least, a thromboplastin having proteolytic activity and a chromogenic substrate for thrombin of the structure H-D-Phe-Pro-Arg-ANBA-R or Tos-Gly-Pro-Arg-ANBA-R, vhere R is NH-$C_nH_{2n+1}$(sic), with n=1 to 7, NH-benzyl or the residue of an amino acid, or its alkyl ester, which are bonded via the α-amino group, and where the substrate is converted by the thromboplastin, in aqueous suspension in the presence of calcium ions, to the extent of less than 0.00125 times the initial concentration per hour at 37°C.

2. A reagent as claimed in claim 1, wherein the thromboplastin is obtained from human placenta.

3. A reagent as claimed in claim 1, wherein the reagent contains calcium ions at a concentration of 0.5 to 10 mmol/l, preferably 5 mmol/l.

4. A reagent as claimed in claim 1, which contains a buffer solution, preferably tris, Hepes, glycylglycine, Epps or imidazole, preferably Hepes, at a concentration of 0.005 to 0.1 mol/l, preferably 0.025 mol/l, with a pH range from 7 to 8.5, preferably 7.6.

5. A reagent as claimed in claim 1, which contains a neutral salt, preferably sodium chloride, at a concentration of 0.01 to 0.2 mol/l, preferably 0.05 mol/l.

6. A reagent as claimed in claim 1, which contains the reagent Polybren® (hexadimethrin bromide/poly-N,N,N',H'-tetramethyl-N-trimethylene hexamethylenediammonium dibromide) in a concentration of 0.01 to 0.5 mg/l, preferably 0.25 mg/l.

7. A reagent as claimed in claim 1, which contains added human or animal factor V.

8. The use of a reagent as claimed in claim 1, with the use of a plasma deficient in factors II, VII, X and V, for the determination of factors II, VII, X and V.

9. The use of a reagent as claimed in claim 7, with the use of a plasma deficient in factors II, VII and X, for the determination of factors II, VII and X.

10. A process for the preparation of a reagent for the photometric determination of the prothrombin time, containing a thromboplastin having proteolytic activity and a chromogenic substrate for thrombin of the structure H-D-Phe-Pro-Arg-ANBA-R or Tos-Gly-Pro-Arg-ANBA-R, where R is NH-$C_nH_{2n+1}$ (sic), with n=1 to 7, NH-benzyl or the residue of an amino acid, or its alkyl ester, which are bonded via the α-amino group, which comprises addition of that chromogenic substrate to a buffer solution with a pH in the range of 7 to 8,5, where the substrate is converted by the thromboplastin, in aqueous suspension in the presence of calcium ions, to the extent of less than 0.00125 times the initial concentration per hour at 37°C, and, where appropriate, the addition of a soluble calcium salt in a concentration of 0.5-10 mmol/l, and, where appropriate, freeze-drying.

11. A method for the photometric determination of the prothrombin time in plasma, which comprises the preparation of serial dilutions of the plasma, addition of a solution which contains a thromboplastin having proteolytic activity, a chromogenic substrate for thrombin of the structure H-D-Phe-Pro-Arg-ANBA-R or Tos-Gly-Pro-Arg-ANBA-R, where R is NH-$C_nH_{2n+1}$ (sic), with n=1 to 7, NH-benzyl or the residue of an amino acid, or its alkyl ester, which are bonded via the α-amino group, and where the substrate is converted by the thromboplastin, in aqueous suspension in the presence of calcium ions, to the extent of less than 0.00125 times the initial concentration per hour at 37°C, and, where appropriate, calcium ions at a concentration of 0.5-10 mmol/l, and determination of the time in which a specified extinction increase is reached.

12. The method as claimed in claim 11, wherein the plasma is diluted with a deficient plasma.

## Revendications

1. Réactif pour la détermination photométrique du temps de thromboplastine, contenant au moins une thromboplasplastine à action protéolytique et un substrat chromogène pour la thrombine, de structure H-D-Phe-Pro-Arg-ANB-R (ANB= acide 5-amino-2-benzoïque) ou Tos-Gly-Pro-Arg-ANB-R, R étant un groupe NH-$C_nH_{2n+1}$ dans lequel n= 1 à 7, NH-benzyle ou le reste d'un acide aminé lié par le groupe α-amino, ou d'un ester alkylique de celui-ci, et le substrat n'étant transformé par la thromboplastine, en suspension aqueuse, en présence d'ions calcium, qu'à raison de moins de 0,00125 fois la concentration initiale, par heure, à 37°C.

2. Réactif selon la revendication 1, caractérisé en ce que l'on obtient la thromboplastine à partir de placenta humain.

3. Réactif selon la revendication 1 , caractérisé en ce que le réactif contient des ions calcium en une concentration de 0,5 à 10 mmoles/litre, de préférence de 5 mmoles/litre.

4. Réactif selon la revendication 1, caractérisé en ce qu'il contient une solution tampon, de préférence Tris, Hepes, glycyl-glycine, Epps, imidazole, de préférence Hepes, en une concentration de 0,005 à 0,1 mole/litre, de préférence de 0,025 mole/litre, ayant un intervalle de pH allant de 7 à 8,5, de préférence un pH égal à 7,6.

5. Réactif selon la revendication 1, caractérisé en ce qu'il contient un sel neutre, de préférence du chlorure de sodium, en une concentration de 0,01 à 0,2 mole/litre, de préférence de 0,05 mole/litre.

6. Réactif selon la revendication 1, caractérisé en ce que le réactif contient du Polybren® (bromure d'hexadiméthrine/poly-N,N,N'-N'-tétraméthyl-N-triméthylène/dibromure d'hexaméthylène-diammonium) en une concentration de 0,01 à 0,5 mg/litre, de préférence de 0,25 mg/litre.

7. Réactif selon la revendication 1, caractérisé en ce qu'il contient du facteur V humain ou animal ajouté.

8. Utilisation d'un réactif selon la revendication 1, avec utilisation d'un plasma déficient en les facteurs II, VII, X et V, pour la détermination des facteurs II, VII, X et V.

9. Utilisation d'un réactif selon la revendication 7, avec utilisation d'un plasma déficient en les facteurs II, VII et X pour la détermination des facteurs II, VII et X.

10. Procédé pour la préparation d'un réactif pour la détermination photométrique du temps de thromboplastine, contenant une thromboplasplastine à action protéolytique et un substrat chromogène pour la thrombine, de structure H-D-Phe-Pro-Arg-ANB-R ou Tos-Gly-Pro-Arg-ANB-R, R étant un groupe $NH-C_nH_{2n+1}$ dans lequel n = 1 à 7, NH-benzyle ou le reste d'un acide aminé lié par le groupe α-amino, ou d'un ester alkylique de celui-ci, caractérisé en ce qu'on ajoute ce substrat chromogène à une solution tampon ayant un intervalle de pH allant de 7 à 8,5, le substrat n'étant transformé par la thromboplastine, en suspension aqueuse, en présence d'ions calcium, qu'à raison de moins de 0,00125 fois la concentration initiale, par heure, à 37°C, ainsi qu'éventuellement un sel de calcium soluble, ajouté en une concentration de 0,5-10 mmoles/litre, et qu'éventuellement on lyophilise.

11. Procédé pour la détermination photométrique du temps de thromboplastine dans du plasma, caractérisé en ce que l'on prépare une série de dilutions du plasma, on y ajoute une solution qui contient une thromboplastine à action protéolytique, un substrat chromogène pour la thrombine, de structure H-D-Phe-Pro-Arg-ANB-R ou Tos-Gly-Pro-Arg-ANB-R, R étant un groupe $NH-C_nH_{2n+1}$ dans lequel n = 1 à 7, NH-benzyle ou le reste d'un acide aminé lié par le groupe α-amino, ou d'un ester alkylique de celui-ci, et le substrat n'étant transformé par la thromboplastine, en suspension aqueuse, en présence d'ions calcium, qu'à raison de moins de 0,00125 fois la concentration initiale, par heure, à 37°C, et éventuellement des ions calcium en une concentration de 0,5-10 mmoles/ litre, et on détermine le temps dans lequel on obtient un accroissement déterminé de l'extinction.

12. Procédé selon la revendication 11, caractérisé en ce que l'on dilue le plasma avec un plasma déficient.